(19) Europäisches Patentamt
European Patent Office
Office européen des brevets

(11) Veröffentlichungsnummer: **0 315 914 A2**

(12) # EUROPÄISCHE PATENTANMELDUNG

(21) Anmeldenummer: **88118410.5**

(22) Anmeldetag: **04.11.88**

(51) Int. Cl.4: **C07C 69/734 , C07C 59/64 , A61K 31/235**

(30) Priorität: **12.11.87 DE 3738407**

(43) Veröffentlichungstag der Anmeldung: **17.05.89 Patentblatt 89/20**

(84) Benannte Vertragsstaaten: **AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Möller, Hinrich, Dr.**
**Haydnstrasse 27**
**D-4019 Monheim(DE)**
Erfinder: **Wallat, Siegfried, Dr.**
**Marie-Curie-Strasse 9**
**D-4019 Monheim(DE)**

(54) **Sebosuppressive topische Zubereitungen.**

(57) Alkyl-arylether-Derivate der Formel (1)

(I) 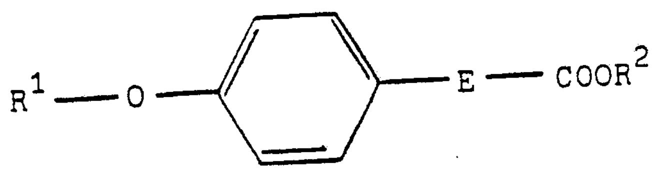

in der $R^1$ eine einfach oder mehrfach verzweigte Alkygruppe mit 8 - 20 C-Atomen, E eine Gruppe $-CH_2-CH_2-$ oder $-CH = CH-$ und $R^2$ eine Alkylgruppe mit 1 - 4 C-Atomen, eine Hydroxyalkylgruppe mit 2 - 4 C-Atomen, eine Alkoxyalkylgruppe, Wasserstoff oder ein salzbildenes Kation ist, eignen sich als sebosuppressive Wirkstoffe in kosmetischen oder pharmazeutischen Zubereitungen zur topischen Anwendung auf dem Haar und auf der Haut.

EP 0 315 914 A2

## Sebosuppressive topische Zubereitungen

Die Erfindung betrifft neue p-Alkoxyarylcarbonsäuren sowie deren Salze und Ester als antiseborrhoische Wirkstoffe und deren Verwendung zur Herstellung von topischen, pharmazeutischen oder kosmetischen Mitteln mit sebosuppressiver Wirkung.

Übermäßige Absonderungen der Talgdrüsen der Oberhaut können zu krankhaften Hautzuständen führen. In häufiger vorkommenden, leichteren Fällen stellen sie ein kosmetisches Problem dar, das sich durch fettiges Aussehen der Haare oder ein glänzendes, öliges Aussehen der Haut manifestiert. Die moderne Kosmetik ist daher bemüht, durch geeignete topisch anwendbare Zubereitungen die Sekretion der Talgdrüsen zu normalisieren und dem Haar und der Haut wieder ein ansprechendes Aussehen zu verleihen.

4-Alkoxy-benzoesäureester sind bereits in den deutschen Patentanmeldungen DE-A 31 21 064, DE-A 35 00 971 und der europäischen Patentanmeldung EP-A 114 051, 4-Alkoxybenzoesäuren und deren Salze in den deutschen Patentanmeldungen DE-A 30 47 106 und DE-A 35 00 972, lineare Alkoxyzimtsäureester in der deutschen Patentanmeldung DE-A 31 21 091 und $C_1$-$C_6$-Alkoxyphenylpropionsäureester in der deutschen Patentanmeldung DE-A 30 47 106 als antiseborrhoische Zusätze für kosmetische Zubereitungen vorgeschlagen worden.

Obwohl die in den genannten Druckschriften vorgeschlagenen Verbindungen eine deutliche antiseborrhoische Wirkung zeigen, besteht doch weiterhin ein Bedürfnis an Zubereitungen mit einer erhöhten Wirksamkeit bei niedrigen Anwendungskonzentrationen. Es wurden nunmehr Verbindungen gefunden, die bereits bei An wendungskonzentrationen von 0,01 Gew.-% und darunter in für topische Applikation geeigneten Trägern eine gute antiseborrhoische Wirkung zeigen.

Gegenstand der Erfindung sind Alkoxyarylcarbonsäuren, deren Salze und Ester der Formel (I)

(I) 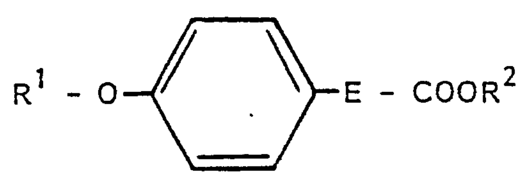

in der $R^1$ eine einfach oder mehrfach verzweigte Alkylgruppe mit 8 bis 20 C-Atomen, E eine Gruppe $-CH_2-CH_2-$ oder $-CH=CH-$ und $R^2$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe, Wasserstoff oder ein salzbildendes Kation ist.

In den Verbindungen der Formel (I) kann $R^1$ z. B. eine 2-Ethylhexyl-, 3,5,5-Trimethylpentyl-( = Isooctyl-), 3,5,5-Trimethylhexyl-( = Isononyl), Isodecyl-, Isotridecyl-, 2-Butyl-octyl-, 2-Hexyldecyl-, Isoheptadecyl-, Isostearyl- oder 2-Octyl-dodecyl-gruppe sein. Die einfach verzweigten Alkylgruppen leiten sich bevorzugt von Alkoholen ab, die durch Aldolkondensation aus Aldehyden und Hydrierung des Aldoladdukts gewonnen werden oder von Alkoholen, die durch Guerbet-Dimerisation aus linearen, primären Alkoholen gewonnen werden. Höher verzweigte Alkylgruppen leiten sich von Alkoholen ab, die durch Oxosynthese (Hydroformylierung) aus verzweigten Olefinen gewonnen werden, die ihrerseits durch Oligomerisation niederer Olefine zugänglich sind, z. B. Diisobutylen, Tripropylen, Tetrapropylen, Triisobutylen, Pentapropylen usw.

$R^2$ ist bevorzugt Wasserstoff oder ein salzbildendes Kation. Besonders geeignet sind dermatologisch verträgliche Salze wie die Alkali- und Erdalkalisalze, z. B. die Natrium-, Kalium-, Calcium-oder Magnesiumsalze, aber auch die Ammonium- und Alkanolammoniumsalze, z. B. das Monoethanolammonium-, das Isopropanolammonium- oder das Triethanolammoniumsalz. Es sind aber auch Salze anderer Basen wirksam, soweit diese ausreichend dermatologisch verträglich sind.

Die p-Alkoxyaryl-carbonsäuren, deren Salze und Ester der Formel (I) sind zwar neu, lassen sich aber als Verbindungen des aus dem genannten Stand der Technik bekannten Grundtyps nach den dort angegebenen literaturbekannten allgemeinen Syntheseverfahren herstellen.

Die p-Alkoxyarylcarbonsäure-methylester werden z. B. durch Alkylierung von p-Hydroxyzimtsäure-methylester oder p-Hydroxyphenylpropionsäure-methylester mit Halogeniden der Formel $R^1$-X (worin X z. B. Chlor oder Brom ist) oder mit entsprechenden Sulfaten oder Sulfonsäureestern hergestellt. Diese sind aus den entsprechenden Alkoholen nach bekannten Literaturverfahren zugänglich.

Die Hydroxyalkyl- und Alkoxyalkylester können aus den entsprechenden p-Alkoxyarylcarbonsäure-methylestern durch Umesterung mit den jeweiligen Alkoholkomponenten $R^2$-OH in Gegenwart alkalischer Katalysatoren, wie z. B. Natriumalkoholaten, hergestellt werden.

Man kann auch umgekehrt zunächst die Veresterung der p-Hydroxyarylcarbonsäuren und danach die Alkylierung durchführen.

Die freien Säuren, in welchen $R^2$ = Wasserstoff ist, lassen sich aus den entsprechenden Methylestern leicht durch Verseifung (Hydrolyse) gewinnen. Durch Neutralisation mit Basen lassen sie sich in die Salze überführen, in welchen $R^2$ das salzbildende Kation ist.

Die p-Alkoxyarylcarbonsäuren, deren Salze und Ester der Formel (I) besitzen eine ausgeprägte sebosuppressive Wirkung, wobei viele der Produkte bereits bei sehr niedrigen Anwendungskonzentrationen eine merkliche sebostatische und antiseborrhoische Wirkung auf der Haut entfalten. Sie besitzen darüber hinaus eine ausgezeichnete Haut- und Schleimhautverträglichkeit. Die Alkoxyarylcarbonsäuren und deren Derivate der Formel (I) werden bevorzugt in einer Menge von 0,0005 bis 2,0 Gew.-% in geeignete Träger eingesetzt. Sie lassen sich problemlos in verschiedene pharmazeutische und kosmetische Träger einarbeiten.

Als kosmetische Träger eignen sich alle für die Aufbringung auf die Haare oder die Haut geeigneten Zubereitungen. Für die Hautbehandlung eignen sich insbesondere wäßrige oder alkoholische Lösungen, tensidhaltige Lotionen, Öle, Salben, Emulsionen, Cremes, Gele und Stiftpräparate. Für die Haarbehandlung eignen sich besonders Haarwässer, Haarshampoos, Haarkuren, Haarspülungen und Haarsprays. Wegen der besonderen kosmetischen Probleme, die durch fettendes Haar verursacht werden, stellen die haarkosmetischen Zubereitungen besonders bevorzugte Ausführungsformen der Erfindung dar.

Die wichtigsten Komponenten üblicher kosmetischer Träger sind

- Ölkomponenten,
z. B. Paraffinöl, Pflanzenöle, Fettsäureester, Squalan, Fettalkohole, 2-Octyldodecanol,
- Fette und Wachse,
z. B. Walrat, Bienenwachs, Montanwachs, Paraffin, Cetyl-stearylalkohol,
- Emulgatoren,
z. B. Fettsäurepartialglyceride, Fettsäure-Sorbitan-partialester und deren Ethoxylate, Seifen, Fettalkoholsulfate, Fettalkoholpolyglycolether, Alkylphosphate,
- Waschrohstoffe,
insbesondere <u>Aniontenside</u>, z. B. Fettalkoholpolyglycolethersulfate, Fettalkoholsulfate, Alphaolefinsulfonate, Alkansulfonate, Sulfobernsteinsäureester, Acyltauride, Acylisethionate und Acylsarkosine,
<u>ampholytische Tenside</u>, z. B. N-Alkylglycin, N-Alkylaminopropionsäure, N-Alkylaminobuttersäure mit 8 bis 18 C-Atomen in der Alkylgruppe, <u>zwitterionische Tenside</u>, z. B. N-Alkyl($C_8$-$C_{18}$)- N,N-dimethylammonioglycinat oder N-Kokosacylaminopropyl-N,N-dimethylammonioglycinat und
<u>nichtionogene Tenside</u>, z. B. Fettalkoholpolyglycolether, Alkylphenolpolyglycolether, Fettsäurepolyglycolester, Aminoxid-Tenside, Fettsäurealkanolamide und deren Ethoxylate und
<u>kationische Tenside</u>, z. B. Alkyl($C_{12}$-$C_{18}$)-trimethylammoniumchlorid, Lauryldimethylbenzylammoniumchlorid, Cetylpyridiniumchlorid, Distearyldimethylammoniumchlorid
- niedere Alkohole wie z. B. Ethanol, Isopropanol,
- mehrwertige Alkohole wie z. B. Ethylenglycol, Propylenglycol, Glycerin,
- Wasser und Hilfsstoffe wie z. B. Duftstoffe, Konservierungsmittel, Puffersubstanzen, Verdickungsmittel, Farbstoffe und Trübungsmittel.

Die folgenden Beispiele sollen den Erfindungsgegenstand näher erläutern:

## <u>Beispiele</u>

### 1. Herstellungsbeispiele

#### 1.1 Isononyloxy-zimtsäure-methylester

Die Mischung aus 14,2 g (79,7 mMol) 4-Hydroxy-zimtsäuremethylester, 150 ml N-Methylpyrrolidon und 14,4 g (79,7 mMol) 30 %ige Natriummethylatlösung wurde nach dem Abdestillieren des Methanols mit 0,5 g Tetrabutylammoniumchlorid und 15,5 g (95,6 mMol) Isononylchlorid versetzt und unter Rühren 8 Stunden auf 160 °C erwärmt. Nach Abkühlen auf Raumtemperatur wurde von gebildetem Natriumchlorid abfiltriert, die Lösung unter vermindertem Druck zur Trockene eingeengt, der Eindampfrückstand in Methylenchlorid

aufgenommen, mit Wasser gewaschen, mit Natriumsulfat getrocknet, nach dem Eindampfen an Kieselgel (Merck) chromatographiert (Elutionsmittel: Methylenchlorid/Toluol = 8 : 2). Es wurden 20,1 g (91 % d. Th.) 4-Isononyloxy-zimtsäure-methylester als farbloses Öl mit einem Brechungsindex n20 : 1,5414 erhalten.

1.2 4-Isotridecyloxy-zimtsäure-methylester Brechungsindex n20 : 1,5334

wurde nach dem in 1.1 beschriebenen Verfahren ausgehend von Isotridecylchlorid hergestellt.

1.3 4-(2-Ethylhexyl)-oxy-zimtsäure-methylester Brechungsindex n20 : 1,5471

wurde nach dem in 1.1 beschriebenen Verfahren ausgehend von 2-Ethylhexyl-chlorid hergestellt.

1.4 3-(4-Isononyloxyphenyl)-propionsäure-methylester (n20: 1,4904)

wurde nach dem in 1.1 beschriebenen Verfahren ausgehend von 3-(4-Hydroxyphenyl)-propionsäuremethylester und Isononylchlorid hergestellt.

1.5 3-(4-Isotridecyloxyphenyl)-propionsäuremethylester (n20 : 1,4902)

wurde nach dem in 1.1 beschriebenen Verfahren ausgehend von 3-(4-hydroxyphenyl)-propionsäuremethylester und Isotridecylchlorid hergestellt.

1.6 4-Isononyloxy-zimtsäure

Die Mischung aus 16 g (52,6 mMol) 4-Isononyloxy-zimtsäuremethylester, 55 ml Ethanol, 40 ml Wasser und 2,5 g Natriumhydroxid wurde 2 Stunden zum Sieden erhitzt, eingedampft, mit Eis/Wasser versetzt und mit 2 m Salzsäure angesäuert. Nach dreimaligem Ausschütteln mit Methylenchlorid, Eindampfen und Umkristallisieren des Rückstandes aus n-Hexan wurde farblose 4-Isononyloxy-zimtsäure vom Schmelzpunkt 128 bis 132 °C in fast quantitativer Ausbeute erhalten.

1.7 4-(2-Ethylhexyl)-oxy-zimtsäure (Schmelzpunkt: 80 bis 81 °C)

wurde nach dem in 1.6 beschriebenen Verfahren ausgehend von dem Methylester (1.3) hergestellt.

1.8 Isotridecyloxy-zimtsäure (Schmelzpunkt: 75 - 91 ° C)

wurde nach dem in 1.6 beschriebenen Verfahren ausgehend von dem Methylester (1.2) hergestellt.

1.9 3-(4-Isotridecyloxyphenyl)-propionsäure (n20 : 1,4990)

wurde nach dem in 1.6 beschriebenen Verfahren ausgehend von dem Methylester (1.5) hergestellt.

1.10 3-(4-Isononyloxyphenyl)-propionsäure (Schmelzpunkt: 63 - 65 ° C)

wurde nach dem in 1.6 beschriebenen Verfahren ausgehend von dem Methylester (1.4) hergestellt.

2. Prüfung und Bewertung der antiseborrhoischen Wirkung

Grundlage

Der Test geht von der Beobachtung aus, daß männliche Ratten ein bräunliches Hautfett absondern, so daß die mehr oder weniger starke Fettigkeit der Haut visuell gut als Hautbräunung beurteilt werden kann. Daß es sich bei der Bräunung um Hautoberflächenfett handelt, ist daran zu erkennen, daß junge weibliche Ratten sowie männliche Ratten nach dem Waschen mit Tensidlösungen oder mit Lipidlösungsmitteln oder auch männliche Ratten, die systematisch mit Östrogen behandelt wurden, nur die normale helle, rosafarbene Haut nach dem Scheren aufweisen. Parallel dazu sind aus den abgeschnittenen Haaren nur noch sehr geringe Lipidmengen zu extrahieren (vgl. hierzu auch J. Soc. Cosmet. Chem. **34**, 127 - 135 (1983)).

Durchführung

Als Versuchstiere dienten männliche Wistar-Ratten mit einem Körpergewicht von 220 bis 230 g zu Versuchsbeginn.

Zur Beurteilung der Wirksamkeit wurden die Prüfsubstanzen in den in Tabelle 1 angegebenen Konzentrationen in Ethanol/Aceton (1 : 1) jeweils 6 Ratten halbseitig auf das Rückenfell gepinselt. Die andere Seite wurde nur mit dem Lösungsmittel behandelt.

Während der Versuchsdauer von 14 Tagen wurde an insgesamt 9 Tagen einmal appliziert. Zur weiteren Kontrolle diente eine Gruppe von 6 Ratten, die beidseitig nur mit dem Lösungsmittel behandelt wurden. Am Ende des Versuchs wurden die Tiere am Rücken und an den Flanken geschoren und von einem Beurteilerpanel (6 Personen) unabhängig unter Doppelblindbedingungen visuell abgemustert. Dabei wurde der Bräunungsgrad auf dem Rücken der Ratten als Maß für den Hautfettbelag visuell beurteilt.

Bewertung

Als 1. Kriterium wurde der Unterschied zwischen rechter und linker Seite gewertet, wobei pro Beurteiler und Tier jeweils 1 Punkt zu vergeben war, und zwar in der Weise, daß die

| | |
|---|---|
| dunklere Seite | mit 1 Punkt |
| hellere Seite | mit 0 Punkten und |
| bei Gleichheit beide Seiten | mit 0,5 Punkten |

benotet wurden.

Signifikante Differenzen zwischen nur mit dem Lösungsmittel behandelter und mit Prüflösung behandelter Seite nach dieser Bewertungsmethode zeigen die lokale Wirksamkeit einer Substanz an.

Als 2. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach dieser Bewertungsmethode werden die Punktsummendifferenzen zwischen den nur mit dem Lösungsmittel behandelten Kontrolltieren und jeweils den behandelten und unbehandelten Seiten ( P) der Versuchstiere gebildet, wobei wiederum signifikante Differenzen zwischen Kontrolltieren und der behandelten Seite der Versuchstiere die Wirkung einer Substanz deutlich machen.

Prozentuale Sebumreduktion

Die Sebumreduktion errechnet sich aus der Punktedifferenz in der Weise, daß man den Quotienten aus der Punktedifferenz P und der Punktezahl für die Kontrollgruppe $P_k$ bildet und den erhaltenen Wert in % angibt.

$$\text{Sebumreduktion} = \frac{P}{P_k} \cdot 100 \quad (\%)$$

Die Ergebnisse für die Substanzen 1.1 bis 1.9 sind der Tabelle I zu entnehmen. Zum Vergleich wurden noch folgende bekannte Verbindungen geprüft (Vergleichssubstanzen, Tabelle II):

| | |
|---|---|
| V 1.11 | 4-n-Decyloxyzimtsäure-methylester |
| V 1.12 | 4-n-Decyloxyphenylpropionsäure-methylester |
| V 1.13 | 4-Isononyloxybenzoesäuremethylester |
| V 1.14 | 4-Isotridecyloxybenzoesäure-methylester |
| V 1.15 | 4-Isotridecyloxyphenylessigsäure-methylester |
| V 1.16 | 4-Isononyloxy-benzoesäure |
| V 1.17 | 4-Isotridecyloxy-benzoesäure |

Tabelle 1

| Produkt gemäß Beispiel | Konzentration Gew.-% | Prozentuale Sebumreduktion |
|---|---|---|
| 1.1 | 0,01 | 46 |
| 1.1 | 0,1 | 98 |
| 1.2 | 0,01 | 28 |
| 1.2 | 0,1 | 100 |
| 1.3 | 0,1 | 31 |
| 1.3 | 0,5 | 67 |
| 1.4 | 0,1 | 100 |
| 1.5 | 0,1 | 100 |
| 1.6 | 0,005 | 35 |
| 1.6 | 0,01 | 72 |
| 1.7 | 0,05 | 24 |
| 1.7 | 0,1 | 82 |
| 1.8 | 0,002 | 20 |
| 1.8 | 0,01 | 82 |
| 1.9 | 0,005 | 23 |
| 1.10 | 0,05 | 55 |

Tabelle II

| (Vergleiche) | | |
|---|---|---|
| Vergleichsprodukt | Konzentration Gew.-% | Prozentuale Sebumreduktion |
| V 1.11 | 0,1 | 11 |
| V 1.12 | 0,05 | 12 |
| V 1.13 | 0,1 | 26 |
| V 1.14 | 0,01 | 0 |
| V 1.15 | 0,1 | 0 |
| V 1.16 | 0,02 | 21 |
| V 1.17. | 0,002 | 0 |

## Ansprüche

1. Alkyl-Arylether-Derivate der Formel (1)

(1) 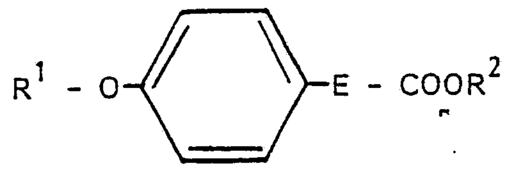

in der $R^1$ eine einfach oder mehrfach verzweigte Alkylgruppe mit 8 bis 20 C-Atomen, E eine Gruppe $-CH_2-CH_2-$ oder $-CH=CH-$ und $R^2$ eine Alkylgruppe mit 1 bis 4 C-Atomen, eine Hydroxyalkylgruppe mit 2 bis 4 C-Atomen, eine Alkoxyalkylgruppe mit 1 bis 4 C-Atomen in der Alkoxy- und 2 bis 4 C-Atomen in der Alkylgruppe, Wasserstoff oder ein salzbildendes Kation is.

2. Verwendung von Alkyl-Arylether-Derivaten der Formel (1) gemäß Anspruch 1 als antiseborrhoische Wirkstoffe zur Herstellung von topischen, pharmazeutischen und kosmetischen Mitteln.

3. Sebosuppressive Zubereitungen zur topischen Anwendung auf dem Haar und auf der Haut, bestehend aus antiseborrhoischen Wirkstoffen in einem dermatologisch verträglichen, kosmetischen oder pharmazeutischen Träger, dadurch gekennzeichnet, daß als antiseborrhoische Wirkstoffe Alkyl-Arylether-Derivate der Formel (1)

(1) 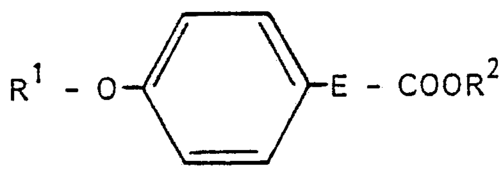

in der $R^1$ eine einfach oder mehrfach verzweigte Alkylgruppe mit 8 bis 20 C-Atomen, E eine Grüppe $-CH_2-CH_2-$ oder $-CH=CH-$ und $R^2$ eine Alkylgruppe mit 1 bis 4 C-Atomen, eine Alkoxyalkylgruppe mit 1 bis 4 C-Atomen in der Alkoxy-und 2 bis 4 C-Atomen in der Alkylgruppe, Wasserstoff oder ein salzbildenes Kation ist.